# EUROPEAN PATENT SPECIFICATION

(11) **EP 3 662 486 B1**
(45) Date of publication and mention of the grant of the patent: **15.04.2026**
(21) Application number: 18749334.1
(22) Date of filing: 27.07.2018
(51) Int. Cl.: G16H 40/60, G16H 50/20

(54) **DEVICE, SYSTEM, AND METHOD FOR DETERMINING WHEN TO SELECT LIMITED ECHOCARDIOGRAPHY EXAMS**
VORRICHTUNG, SYSTEM UND VERFAHREN ZUR BESTIMMUNG DER AUSWAHL VON BEGRENZTEN ECHOKARDIOGRAPHISCHEN UNTERSUCHUNGEN
DISPOSITIF, SYSTEME ET PROCEDE POUR DETERMINER QUAND CHOISIR DES EXAMENS D'ECHOCARDIOGRAPHIE LIMITEE

(30) Priority: 31.07.2017 US 201762538878 P
(43) Date of publication of application: 10.06.2020
(73) Proprietor: Koninklijke Philips N.V., 5656 AG Eindhoven (NL)
(72) Inventor: FORSBERG, Thomas, Andre, 5656 AE Eindhoven (NL); SEVENSTER, Merlijn, 5656 AE Eindhoven (NL); SPENCER, Kirk, 5656 AE Eindhoven (NL)
(74) Representative: Philips Intellectual Property & Standards
(86) International application number: PCT/EP2018/070470
(87) International publication number: WO 2019/025322

(56) References cited:
- US-A- 5 315 999
- US-B1- 6 556 695
- SPENCER KIRK T ET AL: "Focused Cardiac Ultrasound: Recommendations from the American Society of Echocardiography", JOURNAL OF THE AMERICAN SOCIETY OF ECHOCARDIOGRAPHY, MOSBY-YEAR BOOK, INC. ST. LOUIS, MO, US, vol. 26, no. 6, 25 May 2013 (2013-05-25), pages 567 - 581, XP028554509, ISSN: 0894-7317, DOI: 10.1016/J.ECHO.2013.04.001

## Description

### Background Information

A physician may provide healthcare services to patients using a variety of different procedures and recommending a variety of different tests to be performed in pursuit of a proper treatment. For example, the physician may recommend that an examination of a target area on the body of a patient is needed. Accordingly, the physician may recommend that the patient schedule an image acquisition procedure such as an echocardiography (echo) exam. After results of the echo exam are received and possibly after a treatment period, the physician may recommend that the patient schedule a follow up echo exam for the same target area on the body to track a progress of a treatment. In scheduling the image acquisition procedure and any follow up image acquisition procedure, the physician may be a referring physician who provides a respective image order to an image interpreter. The image interpreter may then perform the proper operations in the image acquisition procedure such as selecting the type of echo exam and sequences to be used in capturing images. The captured images may then be used for interpretation to determine results.

The image acquisition procedure may include one or more sequences selected by an image interpreter (e.g., a radiologist) that define how images are to be captured by a technician using image capturing equipment (e.g., echocardiogram machine). Specifically, the sequences may interrogate an anatomy of a desired area under different settings. For example, the settings may be a contrast use, a slice thickness, pulse sequences (for MR scans), etc. Depending on the type of image acquisition procedure (e.g., echo exam) and a sub-type within the selected type of image acquisition procedure (e.g., transthoracic (TTE) or transesophageal (TEE)), there may be predetermined sequences that are to be utilized in capturing images of the patient.

When the image acquisition procedure is an echo exam, the determined sequences may be part of a complete echo exam. The complete echo exam may be used to provide a comprehensive capture of images for a desired area of the patient. The predetermined sequences of the complete echo exam may be independent of the reason for the image acquisition procedure (as may be indicated in the image order from the referring physician) for the comprehensive capture of images such that a complete set of views are generated for the image interpreter. For example, the American Society of Echocardiography (ASE) states that a complete TTE or TEE echo should be acquired for patients. That is, any time that a TTE or TEE echo exam is to be performed, a complete echo exam along with all accompanying predetermined sequences is to be performed.

Although the complete echo exam being comprehensive and generating a complete set of views may provide the most information to the image interpreter, there are scenarios where using all of the predetermined sequences in the complete echo exam may not be the most efficient approach in performing the image acquisition procedure. For example, a patient may have recently had a complete echo exam performed. Thereafter, the referring physician may have provided an image order for a follow up echo exam where the image order specifies a targeted clinical question (e.g., assess pericardial effusion). In view of this limited imaging requirement, another use of the complete echo exam in the follow up echo exam unnecessarily requires the technician to perform sequences to capture images that have no relevance to the targeted clinical question. The above noted scenario of a follow up image acquisition procedure may be only one example where the complete echo exam may be inefficient. The frequency with which these scenarios arise is not inconsequential. For example, a considerable portion of echo exams (e.g., over 12%) are for follow up image acquisition procedures within days (e.g., within one week) of having a complete echo exam.

With associated costs of medical treatments and with considerations from Accountable Care Organizations (ACOs), there is a need to minimize the overall cost of performing image acquisition procedures such as echo exams. Accordingly, a facility (e.g., a hospital) may be under increasing pressure to prove any added value of the services of the facility while keeping costs to a minimum. One manner of minimizing the costs of echo exams is forgoing a complete echo exam and instead using a limited echo exam. A limited echo exam aims to only address a specific clinical question (e.g. pericardial effusion) that is indicated in the image order. Therefore, the limited echo exam needs only acquire and report on a limited number of views and use a corresponding number of sequences rather than the entire list of predetermined sequences of a complete echo exam. With the reduction in a time used by the medical professional (e.g., the image interpreter, the technician, etc.), a time that the patient must spend in the echo exam, and a time in which the echocardiogram equipment is needed, the limited echo exam may be a significant time and cost saver.

Although the limited echo exam may provide significant cost benefits, complete echo exams are still used for various reasons. For example, referring physicians are generally unaware of the circumstances under which a limited echo exam is appropriate for a given patient. Thus, an image order may provide various reasons but the image order may nonetheless instruct that a complete echo exam be performed. Furthermore, even if a limited echo exam is to be included in the image order, the referring physician or the image interpreter may be unaware of the sequences that are to be selected for the limited echo exam. Therefore, there is a need for patient-specific acquisition protocols to determine the appropriate conditions or circumstances under which the limited echo exam is to be used. Such protocols could be useful for minimizing cost. Additionally, there is a further need for the patient-specific acquisition protocols to ensure that the proper sequences are being used to obtain the correct information in the limited echo exam that is necessary and sufficient for a quality diagnosis and addressing the targeted clinical question.

SPENCER KIRK T ET AL: "Focused Cardiac Ultrasound: Recommendations from the American Society of Echocardiography" relates to recommendations for Focused Cardiac Ultrasound (FCU) from the American Society of Echocardiography. It defines FCU as a targeted examination used by a physician to answer specific clinical questions at the bedside, distinguishing it from a comprehensive echocardiogram.

US 6,556,695 B1, "METHOD FOR PRODUCING HIGH RESOLUTION REAL-TIME IMAGES, OF STRUCTURE AND FUNCTION DURING MEDICAL PROCEDURES", Packer et al., relates to a method for combining pre-acquired high-resolution anatomical models with real-time, low-resolution images during a medical procedure. A detailed, dynamic 4D model of an organ like the heart is created beforehand. During the procedure, this high-resolution model is registered with live images (e.g., from an intracardiac ultrasound probe) to provide the physician with a high-quality, large field-of-view display to guide their actions.

US 5,315,999, "ULTRASOUND IMAGING SYSTEM HAVING USER PRESET MODES", Kinicki et al.,relates to an ultrasound imaging system that allows users to create, save, and recall custom imaging presets. A user can adjust imaging parameters while viewing the live image and then save that specific configuration as a preset mode. These presets, which can be tailored for different exam types, patients, or user preferences, can be quickly selected later to ensure consistent and efficient examinations.

### Summary

The exemplary embodiments are directed to a method, comprising: at a workflow server: receiving an image order for a patient, the image order including information indicative of a reason to perform an image acquisition procedure; determining whether the patient has had a prior image acquisition procedure performed within a time threshold relative to the image order; when the prior image acquisition procedure had been performed within the time threshold, determining whether the reason requires a number of views within a view threshold; and when the number of views is within the view threshold, generating an indication that the image acquisition procedure is to be performed with a limited capacity.

The exemplary embodiments are directed to a workflow server, comprising: a transceiver communicating via a communications network, the transceiver configured to receive an image order for a patient, the image order including information indicative of a reason to perform an image acquisition procedure; a memory storing an executable program; and a processor that executes the executable program that causes the processor to perform operations, comprising: determining whether the patient has had a prior image acquisition procedure performed within a time threshold relative to the image order; when the prior image acquisition procedure had been performed within the time threshold, determining whether the reason requires a number of views within a view threshold; and when the number of views is within the view threshold, generating an indication that the image acquisition procedure is to be performed with a limited capacity.

The exemplary embodiments are directed to a method, comprising: at a workflow server: receiving an image order for a patient, the image order including information indicative of a reason to perform an image acquisition procedure; determining whether the patient has had a prior image acquisition procedure performed within a time threshold relative to the image order; when the prior image acquisition procedure had been performed within the time threshold, determining whether the reason requires a number of views within a view threshold; when the number of views is within the view threshold, determining at least one sequence to be used in the image acquisition procedure; and generating an indication that the image acquisition procedure is to be performed with a limited capacity and a further indication including the at least one sequence.

### Brief Description of the Drawings

Fig. 1 shows a system according to the exemplary embodiments.
Fig. 2 shows a workflow server of Fig. 1 according to the exemplary embodiments.
Fig. 3 shows a method for determining whether a complete or limited echocardiography exam is to be used according to the exemplary embodiments.

### Detailed Description

The exemplary embodiments may be further understood with reference to the following description and the related appended drawings, wherein like elements are provided with the same reference numerals. The exemplary embodiments are related to a device, a system, and a method for determining when a limited echocardiography (echo) exam is to be selected for use in an image acquisition procedure. Specifically, the exemplary embodiments are configured to automatically determine when the conditions surrounding an image order from a referring physician warrant the use of the limited echo exam rather than a complete echo exam. As will be described in further detail below, the exemplary embodiments provide a mechanism where the selection determination is performed based on a recent prior echo exam and the clinical question for the currently ordered echo exam. When the limited echo exam is selected, the exemplary embodiments may be configured with a further mechanism that identifies the sequences and corresponding order to be used to generate the proper views for the image interpreter in addressing the clinical question.

It is noted that the exemplary embodiments are described with respect to selecting between a complete echo exam and a limited echo exam based on when a prior echo exam was performed. However, echo exam, the complete or limited versions, and the timing parameter are only exemplary. The exemplary embodiments may be modified to be used with any imaging modality for an image acquisition procedure in which a first version and a second version which is a subset of the first version may be selected for use. The exemplary embodiments may also be modified to utilize any factor or metric in determining between the first and second versions of the imaging modality.

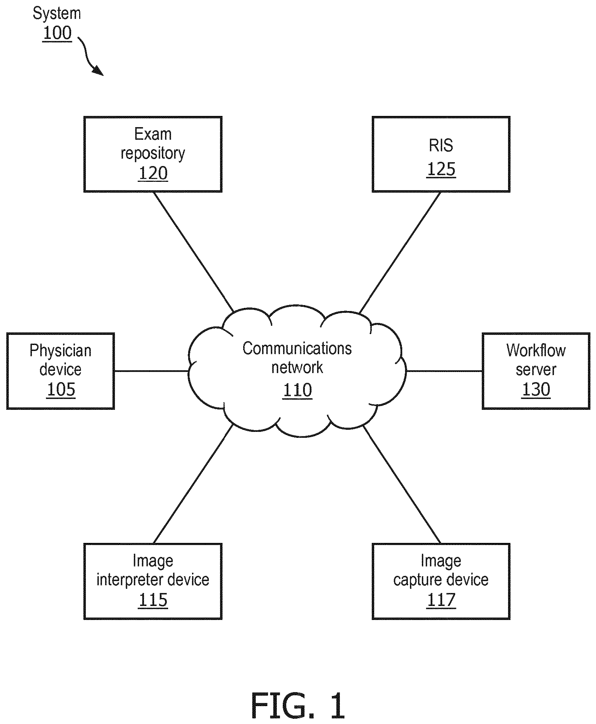

Fig. 1 shows a system 100 according to the exemplary embodiments. The system 100 relates to a communication between various components involved in determining whether to use a complete echo exam or a limited echo exam. Specifically, the system 100 may relate to a scenario when a physician refers the patient to an image interpreter for an echo exam to be performed. However, prior to the image order being created, according to a first exemplary embodiment, the physician may be provided a recommendation of whether the complete or limited echo exam is to be selected based on a determination made according to the exemplary embodiments. According to a second exemplary embodiment, the physician may create the image order which is processed by an image interpreter. The image interpreter or the technician performing the image capture portion of the image acquisition procedure may be provided the recommendation of whether the complete or limited echo exam is to be selected based on the determination made according to the exemplary embodiments. The system 100 may further provide a recommended set of sequences and a corresponding order when the limited echo exam is determined to be used.

It is noted that, as described above, the exemplary embodiments may be configured to generate a recommendation for the referring physician or for the image interpreter/technician. However, for illustrative purposes, the exemplary embodiments are described herein with regard to the recommendation being provided for the referring physician. However, the exemplary embodiments may be modified to provide the recommendation for the image interpreter/technician. With this modification, the exemplary embodiments may utilize a further operation in which the image interpreter/technician queries the referring physician as to whether the recommended selection is to be used. It is also noted that the use of the recommendation is only exemplary. The exemplary embodiments may also be configured with an automatic adoption feature where the determined selection between the complete or limited echo exam is automatically implemented without user intervention.

The system 100 may include a physician device 105, a communications network 110, and an image interpreter device 115. As will be described in further detail below, the system 100 is configured to provide the recommendation for the selection between a complete echo exam and a limited echo exam based on when a previously performed complete echo exam was performed on the same patient. Accordingly, the system 100 may also include an exam repository 120 and a Radiology Information System (RIS) 125. The system 100 may further include a workflow server 130 that utilizes information from these components in determining the proper selection. It is noted that the system 100 may include further components such as sources of data (e.g., a medical data repository including an electronic medical record of the patient) that the workflow server 130 may utilize in determining the proper selection as well as sequences and a corresponding order to perform a limited echo exam when selected for use.

The physician device 105 may represent any electronic device that is configured to perform the functionalities associated with a physician. For example, the physician device 105 may be a portable device such as a tablet, a laptop, etc. or a stationary device such as a desktop terminal. The physician device 105 may include the necessary hardware, software, and/or firmware to perform the various operations associated with medical treatment. The physician device 105 may also include the required connectivity hardware, software, and firmware (e.g., transceiver) to establish a connection with the communications network 110 to further establish a connection with the other components of the system 100.

The physician device 105 may be configured to enable the physician to perform the various operations associated with medical treatment. For example, the physician device 105 may schedule appointments for patients using a calendar application, may track treatments or procedures of a patient, etc. In another example, the physician device 105 may schedule or request an image acquisition procedure to be performed on the patient. More specifically, the physician device 105 may be used to generate the image order that is used for the image acquisition procedure. Subsequently, the patient may determine where and when to have the image acquisition procedure. The physician may enter various inputs to generate the image order. For example, the image order may include information (e.g., target area, expectation of types of information from the results, etc.) that may be used by the image interpreter or technician performing the image acquisition procedure (e.g., selecting a protocol or a sequence to be used). The physician device 105 may utilize any manner of generating the image order. For example, the physician device may use a free form text input, a standardized selection form, or a combination thereof. In view of the physician creating the image order to be used by the image interpreter and the image order indicating whether a limited echo exam is to be used, the exemplary embodiments are configured to provide a recommendation for the physician such that the physician may create the image order at least based on the provided recommendation. In a further example, the physician device 105 may receive test results associated with the image acquisition procedure and display the results to the physician.

The communications network 110 may be configured to communicatively connect the various components of the system 100 to exchange data. The communications network 110 may represent any single or plurality of networks used by the components of the system 100 to communicate with one another. For example, if the physician device 105 is used at a hospital, the communications network 110 may include a private network with which the physician device 105 may initially connect (e.g. a hospital network). The private network may connect to a network of an Internet Service Provider to connect to the Internet. Subsequently, through the Internet, a connection may be established to other electronic devices. For example, the workflow server 130 may be remote relative to the hospital but may be connected to the Internet. Thus, the physician device 105 may be communicatively connected to the workflow server 130. It should be noted that the communications network 110 and all networks that may be included therein may be any type of network. For example, the communications network 110 may be a local area network (LAN), a wide area network (WAN), a virtual LAN (VLAN), a WiFi network, a HotSpot, a cellular network (e.g., 3G, 4G, Long Term Evolution (LTE), etc.), a cloud network, a wired form of these networks, a wireless form of these networks, a combined wired/wireless form of these networks, etc.

The image interpreter device 115 may represent any electronic device that is configured to perform the functionalities associated with an image interpreter. For example, like the physician device 105, the image interpreter device 115 may be a portable device such as a tablet, a laptop, etc. or a stationary device such as a desktop terminal. The image interpreter device 115 may also include the necessary hardware, software, and/or firmware to perform the various operations associated with imaging procedures. The image interpreter device 115 may also include the required connectivity hardware, software, and firmware (e.g., transceiver) to establish a connection with the communications network 110 to further establish a connection with the other components of the system 100.

The image interpreter device 115 may be configured to enable the image interpreter to perform the various operations associated with image acquisition procedures. As those skilled in the art will understand, there are a plurality of different image acquisition procedures that may be performed using different imaging modalities. For example, the image acquisition procedures may be echo exam, an X-ray procedure, a computed tomography (CT) procedure, a magnetic resonance imaging (MRI) procedure, an ultrasound procedure, a positron emission tomography (PET) scan procedure, a single-photon emission computed tomography (SPECT) scan procedure, etc. Although the exemplary embodiments may be used in any of these different modalities, it is again noted that the exemplary embodiments are described with particular regard to echo exams. The image interpreter device 115 may receive an image order from the physician device 105 that indicates whether a complete or limited echo exam is to be performed as well as one or more sequences (with a corresponding order) that is to be used in an image acquisition procedure in which images are captured of a desired area of a patient.

The image capture device 117 may represent the equipment used by a technician in capturing the images for the image acquisition procedure. As noted above, the image capture device 117 may be a specific type of equipment used for a corresponding one of the variety of different types of image acquisition procedures that are available. For example, the image capture device 117 may be an echocardiogram machine, a CT equipment, a MRI equipment, an ultrasound machine, etc. In the exemplary embodiments described herein, the image capture device 117 may be an echocardiogram machine. When the sequences to be used have been determined using the image interpreter device 115, the image capture device 117 may be provided this information so that the technician may perform the appropriate sequences in the proper order to capture the proper images as instructed by the image interpreter. Once the capturing portion of the image acquisition procedure is completed, the image interpreter device 115 may be provided the captured images for interpretation. Using at least one of the captured images, the image interpreter device 115 may be used by the image interpreter to generate test results from the image acquisition procedure which may then be transmitted to the physician device 105.

The exam repository 120 may be a repository of data corresponding to completed image acquisition procedures including completed echo exams. The data may correspond to various aspects of the image acquisition procedure. For example, the data may include the images that are captured, the sequences used in capturing the images, protocols that include a set of sequences that are used, the type of image acquisition procedure that was performed, etc. In this manner, the exam repository 120 may be substantially similar to a Picture Archiving and Communication System (PACS). As those skilled in the art will understand, the PACS may be a system that tracks and logs sequences that are used in image acquisition procedures. Accordingly, data related to selected sequences as determined by the image interpreter using the image interpreter device 115 and sequences actually used by the technician using the image capture device 117 may be received by the PACS to perform this functionality. In this manner, imaging studies of image acquisition procedures may be consumed to track the sequences that have been used to capture images, to track the images opened for interpretation, etc. The exam repository 120 may be configured with these features of the PACS.

The RIS 125 may be a system that electronically manages imaging related details. For example, a hospital or other medical facility may establish the RIS 125 to track the imaging related details. For example, the RIS 125 may track when patients are or have been scheduled for image acquisition procedures including echo exams, how resources are being managed at the medical facility such as how modality equipment is being used, when stages of the image acquisition procedure are being performed including image capture and image interpretation, when results of an image acquisition procedure have been generated and/or provided to the referring physician, etc. as well as administrative aspects such as billing. Accordingly, with regard to the exemplary embodiments, the RIS 125 may track when echo exams and sequences used in the echo exams were performed as well as track when the resulting images were created. The RIS 125 may also track the reason included in the image order to schedule the echo exam.

It is noted that the system 100 including the RIS 125 is only exemplary. As will become apparent below, the exemplary embodiments may utilize the RIS 125 for timing information. That is, the workflow server 130 may receive the timing information from the RIS 125 since the RIS 125 provides a scheduling database or other timing information with respect to when echo exams are performed and associated information (e.g., whether the echo exam was complete or limited, the types of sequences that were used, etc.). However, according to another exemplary embodiment, the exam repository 120 may also be configured to store timing information. For example, images that are captured may have an associated timestamp. Using this timestamp, timing information may be determined. Therefore, the RIS 125 may provide direct timing information of when echo exams were performed but is not necessary as the timing information may still be determined based on other available timing information.

It is also noted that the system 100 may include further components that provide information to the workflow server 130 to provide the features according to the exemplary embodiments. For example, the system 100 may include a medical data repository that stores medical data pertaining to patients. For example, the medical data repository may be directed to patient histories where each patient may have an electronic medical record (EMR) used to track the different procedures, treatments, visits, etc. of the patient. In this manner, the medical data repository may include patient information and a patient identifier related to a select patient. The patient identifier may be associated with the images that are captured and tracked in the exam repository 120 as well as the imaging related details stored in the RIS 125.

It is further noted that the system 100 may include various normalizing components or the components may incorporate a normalizing feature. As those skilled in the art will understand, information from the physician device 105, the image interpreter device 115, and/or the image capture device 117 may be received in various formats. For example, the formats may include a selection form, a narrative report, or a combination thereof. Within the various formats, there may also be different types of information that may be included such as free text or International Classification of Diseases (ICD) codes. In view of the different manners that information may be gathered by the components of the system 100, the normalizing aspect may be used to create a standardized format to be used for the gathered information for consistency across all the sources of information.

The workflow server 130 may be a component of the system 100 that performs functionalities associated with determining whether an echo exam is to be scheduled in an image order as complete or limited. The functionalities may also be associated with determining the sequences to be used when a limited echo exam is selected. Thus, as will be described in further detail below, the workflow server 130 may include a first mechanism in which timing and characteristic information of past image acquisition procedures (particularly complete echo exams) is used to determine whether a subsequent image acquisition procedure is to be complete or limited. The workflow server 130 may include a second mechanism in which sequences are selected to perform the limited echo exam so that the desired results may be captured in the images. It is noted that since a complete echo exam is already configured with a predetermined set of sequences, no determination of which sequences to select is required.

It is noted that the system 100 may include a plurality of physician devices 105, a plurality of image interpreter devices 115, a plurality of image capture devices 117, and a plurality of workflow servers 130. That is, many different physicians and image interpreters may utilize the system 100. There may also be many different workflow servers 130 that service different physician devices 105 and image interpreter devices 115. The exam repository 120 and the RIS 130 may also be systems including a plurality of different components.

It is also noted that the functionalities of the exam repository 120 and the RIS 130 being implemented in separate components of the system 100 is only exemplary. According to another exemplary embodiment, the workflow server 130 may incorporate the functionalities of the exam repository 120 and the RIS 130. In such a scenario, the workflow server 130 may include the required storage components to properly store the data for subsequent use.

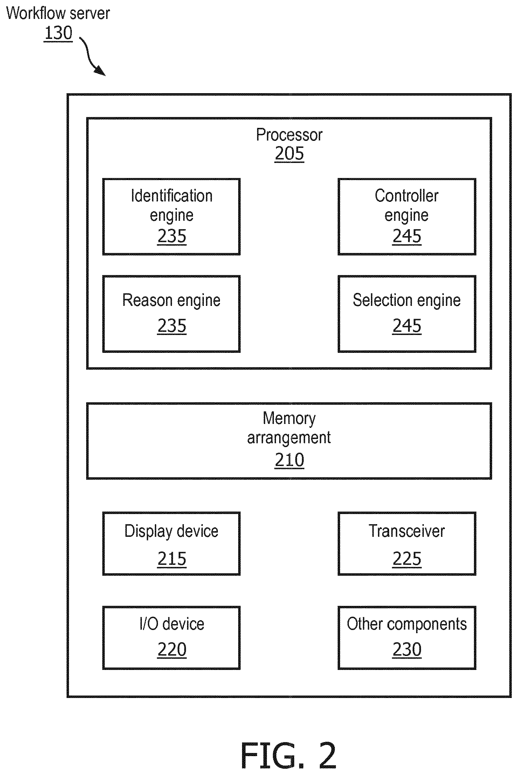

As described above, the workflow server 130 may perform the first mechanism of determining which type of echo exam to select and the second mechanism of determining the sequences to be used when a limited echo exam is selected. Fig. 2 shows the workflow server 130 of Fig. 1 according to the exemplary embodiments. The workflow server 130 may provide various functionalities in determining the type of echo exam to be used in an image acquisition procedure. Although the workflow server 130 is described as a network component (specifically a server), the workflow server 130 may be embodied in a variety of hardware components such as a portable device (e.g., a tablet, a smartphone, a laptop, etc.), a stationary device (e.g., a desktop terminal), incorporated into the physician device 105 and/or the image interpreter device 115, incorporated into a website service, incorporated as a cloud device, etc. The workflow server 130 may include a processor 205, a memory arrangement 210, a display device 215, an input and output (I/O) device 220, a transceiver 225, and other components 230 (e.g., an imager, an audio I/O device, a battery, a data acquisition device, ports to electrically connect the workflow server 130 to other electronic devices, etc.).

The processor 205 may be configured to execute a plurality of applications of the workflow server 130. Specifically, the processor 205 may utilize a plurality of engines including an identification engine 235, a reason engine 240, a controller engine 245, and a selection engine 250. Based on outputs from these engines 235-250, the workflow server 130 may provide the proper recommendation to the physician device 105 along with any further recommendations. The memory 210 may be a hardware component configured to store data related to operations performed by the workflow server 130. Specifically, the memory 210 may store data related to the engines 235-250 such as the identified information and associated information of prior echo exams. The display device 215 may be a hardware component configured to show data to a user while the I/O device 220 may be a hardware component that enables the user to enter inputs. For example, an administrator of the workflow server 130 may maintain and update the functionalities of the workflow server 130 through user interfaces shown on the display device 215 with inputs entered with the I/O device 220 (e.g., the value of the time threshold that a prior echo exam must satisfy). It should be noted that the display device 215 and the I/O device 220 may be separate components or integrated together such as a touchscreen. The transceiver 225 may be a hardware component configured to transmit and/or receive data via the communications network 110.

It should be noted that the above noted applications and engines each being an application (e.g., a program) executed by the processor 205 is only exemplary. The functionality associated with the applications may also be represented as components of one or more multifunctional programs, a separate incorporated component of the workflow server 130 or may be a modular component coupled to the workflow server 130, e.g., an integrated circuit with or without firmware.

The identification engine 235 may be utilized for various identification purposes. In a first identification, the identification engine 235 may determine an identity of the patient. For example, while the physician device 105 is being used to create the image order, the name of the patient or the patient identifier may have been entered by the physician. Based on this identity of the patient, the identification engine 235 may perform a further operation. Specifically, in a second identification, the identification engine 235 may determine a prior echo exam that was performed on the patient. As noted above, the RIS 125 may include scheduling information or the images in the exam repository 120 may include time stamps. Accordingly, the identification engine 235 may utilize this timing information to determine the prior echo exam that was performed. In a first example, the identification engine 235 may determine the most recent echo exam that was performed. In a second example, the identification engine 235 may determine prior echo exams that satisfy a time threshold (e.g., within one week from the currently pending image order). It is noted that the time threshold may be any acceptable value where a prior echo exam has produced images that are still viable for use in interpretation. In fact, depending on the clinical question (to be discussed below), the time threshold may be dynamically selected from a predetermined table of time thresholds with corresponding sub-anatomies. In a third identification, the identification engine 235 may determine the type of the prior echo exam that was performed (e.g., complete or limited), the sequences used in the prior echo exam, etc. That is, the characteristics of the prior echo exam may be determined.

The reason engine 240 may be utilized for determining characteristics of the currently pending image order. For example, as provided by the physician, the currently pending image order may be for a follow up echo exam in a targeted area. Therefore, the currently pending image order may not require as extensive of an image acquisition procedure as a previously performed complete echo exam. However, if the currently pending image order is for an entirely separate reason from any prior echo exam, the currently pending image order may again require that the complete echo exam be used.

As described above, the referring physician may enter various inputs in creating an image order. For example, the image order may include a standardized code (e.g., an ICD10 code) and may also be complimented with free text (e.g., further description provided by the referring physician in narrative form). Thus, in a particular exemplary implementation, the reason engine 240 may output a data structure that comprises a list of anatomies or sub-anatomies to which the currently pending image order will relate. For example, a list of cardiac sub-anatomies that are to be assessed per the image order may be determined. The reason engine 240 may utilize any mechanism in determining the reason, clinical question, and associated list of anatomies/sub-anatomies to which the currently pending image order relates. For example, the reason engine 240 may use parse the image order using natural language processing (NLP) techniques to extract the clinical question. It is noted that the reason engine 240 may be configured with a reasonable assumption that vocabulary used in describing a clinical question in an image order is relatively limited and may be recognized via existing NLP techniques (e.g., regular expressions).

The clinical question or reason in the currently pending image order may indicate a particular region that is to be assessed or eliminate certain regions from having to be assessed. For example, the currently pending image order may be to assess the left ventricle. In such a case, the reason engine 240 may extract the relevant cardiac sub-anatomy (e.g., LV) mentioned in the clinical question. In furtherance of this exemplary implementation, the reason engine 240 may leverage various techniques so that the anatomies/sub-anatomies may be mapped onto anatomy-concepts in a medical ontology (e.g., SNOMED). In another example, the currently pending image order may include a clinical question that is phrased to indicate a status of a certain disease (e.g., "assess left ventricular hypertrophy") or clinical diagnosis (e.g., "rule out mitral valve disease"). In such a scenario, the reason engine 240 may map diagnoses onto a sub-anatomy (e.g., "rule out mitral valve disease" may equate to mitral valve as well as omission of any need for assessment). These mappings may be stored in a look up table (e.g., an anatomy-view lookup table) or the relationships of the medical ontology may be used for the same purpose.

The controller engine 245 may be utilized for outputting various results from comparing the outputs of the identification engine 235 and the reason engine 240. In an initial operation, the controller engine 245 may be configured to map sub-anatomies to views. For example, the controller engine 245 may map a mitral valve to a parasternal long axis (PLAX), an apical four chamber (AP4), an apical two chamber (AP2), etc. In another example, the controller engine 245 may map a pericardium to a subcostal (SC). In this manner, the controller engine 245 may generate a table with these mappings. Therefore, the table may include a complete list of mappings. The table may be generic or configured to fit a medical facility. It is noted that the mapping may be included in a predetermined table that is available to the workflow server 130. Accordingly, the controller engine 245 may have access (e.g., stored in the memory 210) to the table for subsequent use.

As noted above, the controller engine 245 may use the outputs from the identification engine 235 and the reason engine 240. Specifically, by receiving the information corresponding to the currently pending image order, the controller engine 245 may confirm that at least one prior echo exam satisfying the time threshold is available from the identification engine 245 for the particular patient in question. Subsequently, if there is at least one prior echo exam that satisfies the time threshold, the controller engine 245 may determine how the clinical question/reason for the currently pending image order may be properly assessed with a subsequent echo exam. Specifically, based on the results of the reason engine 240 and mapping the resulting cardiac anatomies that are leveraged onto the table, the controller engine 245 may determine whether a net list of views is indeed limited based on the complete list of the table.

The selection engine 250 may be utilized for determining the proper recommendation for the currently pending image order. Specifically, based on the results of the controller engine 245, the selection engine 250 may determine whether the currently pending image order should be for a complete echo exam or a limited echo exam. In a particular exemplary embodiment, the selection engine 250 may use a view threshold that defines a maximum percentage of views produced from a complete echo exam. Thus, if the clinical question/reason only requires up to the percentage of the view threshold, then the selection engine 250 may determine that the limited echo exam should be selected. In contrast, if the percentage of the views of the complete echo exam exceeds the view threshold, the selection engine 250 may determine that the complete echo exam should instead be selected. For example, the view threshold may be whether the required views for the clinical question/reason of the currently pending image order is 30% of the average number of views produced in a complete echo exam. When returning the recommendation for the type of echo exam to be used, the selection engine 250 may also provide evidence to the referring physician that motivates the reasoning for selecting the indicated type of echo exam.

As noted above, the workflow server 130 may also generate a recommendation of the sequences to be used when a limited echo exam is selected for recommendation. Thus, based on the mapped anatomies from the reason engine 240, the selection engine 250 may also determine the corresponding sequences that are to be used in the limited echo exam. For example, as noted above, the clinical question may indicate assessing the pericardium. Therefore, the mapping to the SC may be used as the basis of identifying the corresponding sequences for the SC. In this manner, the referring physician may also be provided the recommended sequences.

It is noted that the recommended sequences being provided to the referring physician is only exemplary. As the sequences are usually selected by the image interpreter and used by the technician, the workflow server 130 may be configured to provide the recommended sequences to these professionals (via the image interpreter device 115 and/or the image capture device 117). Thus, prior to the image interpreter determining the sequences for an image acquisition procedure as indicated in the image order from the referring physician (who has indicated that a limited echo exam is to be used) or prior to the technician beginning the image capture portion of the image acquisition procedure, the workflow server 130 may provide these recommended sequences that correspond to the limited echo exam.

The exemplary embodiments are described above with regard to a recommendation being provided to the physician using the physician device 105. However, the exemplary embodiments may be modified to provide the recommendation to the image interpreter using the image interpreter device 115 or the technician using the image capture device 117. Specifically, the physician may have created the image order based on any available information. However, upon receiving the image order, the image interpreter or the technician may receive a recommendation from the workflow server 130 that indicates a different echo exam to be used than what is indicated in the image order (e.g., image order indicates a complete echo exam while the recommendation indicates a limited echo exam, or vice versa). The image interpreter or technician may contact the referring physician issuing the image order to verify whether the other type of echo exam may be performed. Based on the response from the referring physician, the image interpreter or technician may then proceed accordingly with the image acquisition procedure. In another exemplary implementation, the recommendation from the workflow server 130 may be received by the image interpreter device or the image capture device 117 and be implemented in an automated manner.

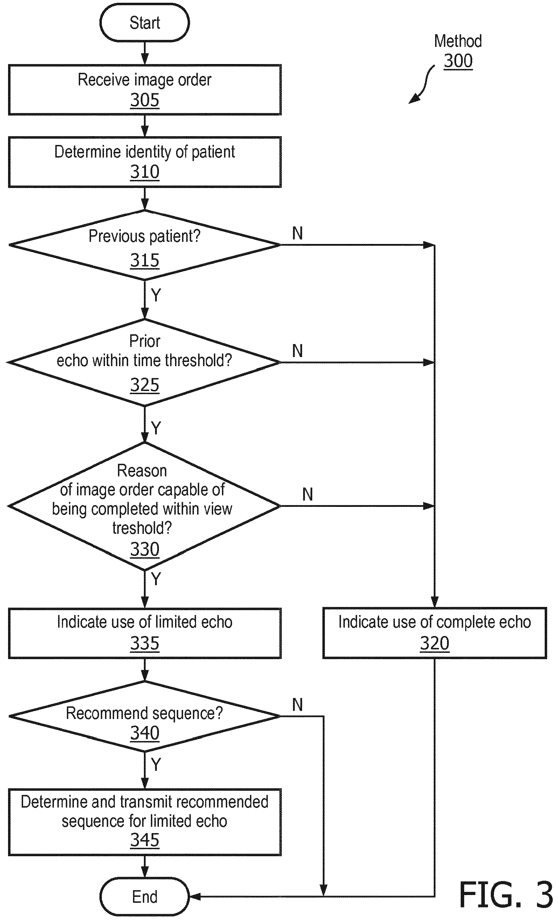

Fig. 3 shows a method 300 for determining whether a complete or limited echo exam is to be used according to the exemplary embodiments. Specifically, the method 300 may relate to the first mechanism of the exemplary embodiments in which the identification engine 235, the reason engine 240, the controller engine 245, and the selection engine 250 determine the type of echo exam to be used based on timing of prior echo exams and the clinical question of a currently pending image order. The method 300 may also incorporate the second mechanism of determining recommended sequences to be used if a limited echo exam is selected. The method 300 will be described from the perspective of the workflow server 130. The method 300 will also be described with regard to the system 100 of Fig. 1 and the workflow server 130 of Fig. 2.

In 305, the workflow server 130 receives an image order. Specifically, the image order may represent the information for a currently pending image order that is being drafted by a referring physician on behalf of a patient. Accordingly, the information may include various types of information such as the identity of the patient, the reason or clinical question behind the image order, etc. In 310, the workflow server 130 determines the identity of the patient. For example, the information may include the name entered with text or use a patient identifier.

In 315, the workflow server 130 determines whether the patient is a previous patient. Specifically, the workflow server 130 may use the exam repository 120, the RIS 125, and/or other sources (e.g., a medical data repository) to determine if there is any previously stored data corresponding to the identified patient in the image order. If there is not previously stored data and the patient is new, the workflow server 130 may determine that there will not be any prior echo exams for the patient. Thus, the workflow server 130 continues to 320 where the workflow server 130 generates an indication that the complete echo exam should be used and transmit the indication to the referring physician.

If the patient has some previously stored data, the workflow server 130 continues from 315 to 325. In 325, the workflow server 130 determines whether there have been any prior echo exams within a predetermined time threshold. For example, the workflow server 130 may identify any prior echo exams that were performed within a week of the currently pending image order. It is again noted that the predetermined time threshold may be static, dynamic, etc. based on various factors including the clinical question or target anatomy of the currently pending image order. If there is no prior echo exam, the workflow server 130 again continues to 320 where a complete echo exam is recommended.

If the previous patient has at least one prior echo exam that is within the time threshold, the workflow server 130 continues from 325 to 330. In 330, the workflow server 130 determines whether the clinical question/reason for the currently pending image order may be capable of being completed with views that are within a predetermined view threshold. For example, the image order may include an ICD-10 that identifies the reason. As described above, the view threshold may be a percentage of the average views captured in images from performing a complete echo exam. Thus, if the reason for the currently pending image order requires a number of views that exceed the view threshold, the workflow server again continues to 320 where a complete echo exam is recommended.

If the number of views for the reason in the currently pending image order requires a number of views that is within the view threshold, the workflow server 130 continues from 330 to 335. In 335, the workflow server 130 generates an indication that the limited echo exam should be used and transmit the indication to the referring physician (along with any further information such as the reasoning for this selection).

If the limited echo exam is determined for selection, in 340, the workflow server 130 determines whether one or more sequences for the limited echo exam is to be recommended. As described above, the mapping of the anatomies from the clinical question included in the currently pending image order may provide insight as to the views that are needed. The corresponding sequences for these views may be identified and recommended. If the sequences are to be manually selected by the image interpreter, then the workflow server 130 may end the method 300. However, fi the sequences are to be recommended to the referring physician, the image interpreter, or the technician, in 345, the workflow server 130 determines and transmits the recommended sequence(s) for the limited echo exam.

It is noted that the above description relates to when the prior echo exam is a complete echo exam. However, the workflow server 130 may be configured to determine whether the prior echo exam is either a complete echo exam or a limited echo exam. If the prior echo exam is a complete echo exam that also satisfies the time threshold, the workflow server 130 may proceed as described above. However, if the prior echo exam is a limited echo exam that also satisfies the time threshold, the workflow server 130 may initially determine whether there is any prior echo exam that is a complete echo exam that satisfies the time threshold. Accordingly, the workflow server 130 may be configured to recommend the limited echo exam only when a prior complete echo exam has been identified to be within the time threshold. However, it is noted that this condition is only exemplary and the workflow server 130 may base the recommendation using any prior echo exam.

The exemplary embodiments provide a device, system, and method of determining whether an image order for an image acquisition procedure may include an instruction to perform a subset of sequences compared to a full set of sequences. In a specific implementation, the image acquisition procedure may be an echo exam where the full set of sequences is for a complete echo exam where a subset of the sequences is for a limited echo exam. When a prior echo exam that satisfies a time threshold is identified, a view threshold for a number of required views for a clinical question/reason in the image order may be assessed with a view threshold. When the view threshold is also satisfied, the limited echo exam may provide sufficient results for the referring physician without the need for the complete echo exam and the superfluous sequences that would be used to create images for unnecessary views.

Those skilled in the art will understand that the above-described exemplary embodiments may be implemented in any suitable software or hardware configuration or combination thereof. An exemplary hardware platform for implementing the exemplary embodiments may include, for example, an Intel x86 based platform with compatible operating system, a Windows platform, a Mac platform and MAC OS, a mobile device having an operating system such as iOS, Android, etc. In a further example, the exemplary embodiments of the above described method may be embodied as a computer program product containing lines of code stored on a computer readable storage medium that may be executed on a processor or microprocessor. The storage medium may be, for example, a local or remote data repository compatible or formatted for use with the above noted operating systems using any storage operation.

## Claims

1. A method, comprising:
at a workflow server:
receiving an image order for a patient, the image order including information indicative of a reason to perform an image acquisition procedure, wherein the image acquisition procedure is either one of a full or a limited image acquisition procedure, wherein the full image acquisition procedure acquires a greater number of views than the limited acquisition procedure;
determining whether the patient has had a prior full image acquisition procedure performed within a time threshold relative to the image order;
when the prior full image acquisition procedure had been performed within the time threshold, determining whether the reason requires a number of views within a view threshold; and
when the number of views is within the view threshold, generating an indication that the image acquisition procedure is to be performed as a limited image acquisition procedure.

2. The method of claim 1, wherein the image acquisition procedure corresponds to image capturing operations performed with an echocardiography exam, wherein the echocardiography exam is one of a complete echocardiography exam or a limited echocardiography exam.

3. The method of claim 2, wherein the complete echocardiography exam has an average number of views that are created, wherein the view threshold is a percentage of the average number of views.

4. The method of claim 3, wherein the view threshold is 30% of the average number of views.

5. The method of claim 1, wherein the time threshold is 1 week.

6. The method of claim 1, further comprising:
when the image acquisition procedure is to be performed as the limited image acquisition procedure, determining at least one sequence of an echocardiography examination to be used in the image acquisition procedure; and
generating a further indication including the at least one sequence.

7. The method of claim 6, wherein the at least one sequence is determined based on the reason and a mapping of an anatomy of the reason to a corresponding view.

8. The method of claim 1, further comprising:
transmitting the indication to one of a physician device, an image interpreter device, an image capture device, or a combination thereof.

9. A workflow server, comprising:
a transceiver communicating via a communications network, the transceiver configured to receive an image order for a patient, the image order including information indicative of a reason to perform an image acquisition procedure, wherein the image acquisition procedure is either one of a full or a limited image acquisition procedure, wherein the full image acquisition procedure acquires a greater number of views than the limited acquisition procedure;
a memory storing an executable program; and
a processor that executes the executable program that causes the processor to perform operations, comprising:
determining whether the patient has had a prior full image acquisition procedure performed within a time threshold relative to the image order;
when the prior full image acquisition procedure had been performed within the time threshold, determining whether the reason requires a number of views within a view threshold; and
when the number of views is within the view threshold, generating an indication that the image acquisition procedure is to be performed as a limited image acquisition procedure.

10. The workflow server of claim 9, wherein the image acquisition procedure corresponds to image capturing operations performed with an echocardiography exam, wherein the echocardiography exam is one of a complete echocardiography exam or a limited echocardiography exam.

11. The workflow server of claim 10, wherein the complete echocardiography exam has an average number of views that are createdwherein the view threshold is a percentage of the average number of views.

12. The workflow server of claim 11, wherein the view threshold is 30% of the average number of views.

13. The workflow server of claim 9, wherein the time threshold is 1 week.

14. The workflow server of claim 9, wherein the operations of the processor further comprise:
when the image acquisition procedure is to be performed as the limited image acquisition procedure, determining at least one sequence of an echocardiography examination to be used in the image acquisition procedure; and
generating a further indication including the at least one sequence.

15. The workflow server of claim 14, wherein the at least one sequence is determined based on the reason and a mapping of an anatomy of the reason to a corresponding view.

## Patentansprüche

1. Verfahren, umfassend:
auf einem Workflow-Server:
Empfangen eines Bildauftrags für einen Patienten, wobei der Bildauftrag Informationen einschließt, die auf einen Grund zum Durchführen eines Bilderfassungsvorgangs hinweisen, wobei der Bilderfassungsvorgang entweder ein vollständiger oder ein eingeschränkter Bilderfassungsvorgang ist, wobei der vollständige Bilderfassungsvorgang eine größere Anzahl von Ansichten erfasst als der eingeschränkte Bilderfassungsvorgang;
Feststellen, ob bei dem Patienten innerhalb eines Zeitschwellenwertes im Verhältnis zum Bildauftrag ein vorheriger vollständiger Bilderfassungsvorgang durchgeführt worden war;
wenn der vorherige vollständige Bilderfassungsvorgang innerhalb des Zeitschwellenwertes durchgeführt worden war, Feststellen, ob der Grund eine Anzahl von Ansichten innerhalb eines Ansichtsschwellenwertes erfordert; und
wenn die Anzahl von Ansichten innerhalb des Ansichtsschwellenwertes liegt, Erzeugen einer Anzeige, dass das Bilderfassungsvorgang als ein begrenzter Bilderfassungsvorgang durchgeführt werden soll.

2. Verfahren nach Anspruch 1, wobei der Bilderfassungsvorgang Bildaufnahmeoperationen entspricht, die bei einer Echokardiographieuntersuchung durchgeführt werden, wobei die Echokardiographieuntersuchung entweder eine vollständige Echokardiographieuntersuchung oder eine eingeschränkte Echokardiographieuntersuchung ist.

3. Verfahren nach Anspruch 2, wobei die vollständige Echokardiographieuntersuchung eine durchschnittliche Anzahl von Ansichten aufweist, die erzeugt werden, wobei der Ansichtsschwellenwert ein Prozentsatz der durchschnittlichen Anzahl von Ansichten ist.

4. Verfahren nach Anspruch 3, wobei der Ansichtsschwellenwert 30 % der durchschnittlichen Anzahl von Ansichten beträgt.

5. Verfahren nach Anspruch 1, wobei der Zeitschwellenwert 1 Woche beträgt.

6. Verfahren nach Anspruch 1, weiter umfassend:
wenn der Bilderfassungsvorgang als der eingeschränkte Bilderfassungsvorgang durchgeführt werden soll, Bestimmen mindestens einer Sequenz einer Echokardiographieuntersuchung, die im Bilderfassungsvorgang verwendet werden soll; und
Erzeugen einer weiteren Anzeige, welche die mindestens eine Sequenz einschließt.

7. Verfahren nach Anspruch 6, wobei die mindestens eine Sequenz auf der Grundlage des Grundes und einer Zuordnung einer Anatomie des Grundes zu einer entsprechenden Ansicht bestimmt wird.

8. Verfahren nach Anspruch 1, weiter umfassend:
Übermitteln der Anzeige an eines von einer Arztvorrichtung, einer Bildinterpretationsvorrichtung, einer Bildaufnahmevorrichtung oder einer Kombination davon.

9. Workflow-Server, umfassend:
einen Transceiver, der über ein Kommunikationsnetz kommuniziert, wobei der Transceiver so konfiguriert ist, dass er einen Bildauftrag für einen Patienten empfängt, wobei der Bildauftrag Informationen einschließt, die auf einen Grund zum Durchführen eines Bilderfassungsvorgangs hinweisen, wobei der Bilderfassungsvorgang entweder ein vollständiger oder ein eingeschränkter Bilderfassungsvorgang ist, wobei der vollständige Bilderfassungsvorgang eine größere Anzahl von Ansichten erfasst als der eingeschränkte Bilderfassungsvorgang;
einen Speicher, der ein ausführbares Programm speichert; und
einen Prozessor, der das ausführbare Programm ausführt, das den Prozessor veranlasst, Operationen durchzuführen, die Folgendes umfassen:
Feststellen, ob bei dem Patienten innerhalb eines Zeitschwellenwertes im Verhältnis zum Bildauftrag ein vorheriger vollständiger Bilderfassungsvorgang durchgeführt worden war;
wenn der vorherige vollständige Bilderfassungsvorgang innerhalb des Zeitschwellenwertes durchgeführt worden war, Feststellen, ob der Grund eine Anzahl von Ansichten innerhalb eines Ansichtsschwellenwertes erfordert; und
wenn die Anzahl von Ansichten innerhalb des Ansichtsschwellenwertes liegt, Erzeugen einer Anzeige, dass das Bilderfassungsvorgang als ein begrenzter Bilderfassungsvorgang durchgeführt werden soll.

10. Workflow-Server nach Anspruch 9, wobei der Bilderfassungsvorgang Bildaufnahmeoperationen entspricht, die bei einer Echokardiographieuntersuchung durchgeführt werden, wobei die Echokardiographieuntersuchung entweder eine vollständige Echokardiographieuntersuchung oder eine eingeschränkte Echokardiographieuntersuchung ist.

11. Workflow-Server nach Anspruch 10, wobei die vollständige Echokardiographieuntersuchung eine durchschnittliche Anzahl von Ansichten aufweist, die erzeugt werden, wobei der Ansichtsschwellenwert ein Prozentsatz der durchschnittlichen Anzahl von Ansichten ist.

12. Workflow-Server nach Anspruch 11, wobei Ansichtsschwellenwert 30 % der durchschnittlichen Anzahl von Ansichten beträgt.

13. Workflow-Server nach Anspruch 9, wobei der Zeitschwellenwert 1 Woche beträgt.

14. Workflow-Server nach Anspruch 9, wobei die Operationen des Prozessors weiter Folgendes umfassen:
wenn der Bilderfassungsvorgang als der eingeschränkte Bilderfassungsvorgang durchgeführt werden soll, Bestimmen mindestens einer Sequenz einer Echokardiographieuntersuchung, die im Bilderfassungsvorgang verwendet werden soll; und
Erzeugen einer weiteren Anzeige, welche die mindestens eine Sequenz einschließt.

15. Workflow-Server nach Anspruch 14, wobei die mindestens eine Sequenz auf der Grundlage des Grundes und einer Zuordnung einer Anatomie des Grundes zu einer entsprechenden Ansicht bestimmt wird.

## Revendications

1. Procédé, comprenant :
sur un serveur de flux de travail :
la réception d'une demande d'examen d'imagerie pour un patient, la demande d'examen d'imagerie incluant des informations indiquant une raison de réaliser une procédure d'acquisition d'images, dans laquelle la procédure d'acquisition d'images est soit une procédure d'acquisition d'images complète, soit une procédure d'acquisition d'images limitée, dans laquelle la procédure d'acquisition d'images complète permet d'obtenir un plus grand nombre de vues que la procédure d'acquisition limitée ;
la détermination permettant de savoir si le patient a déjà subi une procédure d'acquisition d'images complète réalisée dans un délai donné par rapport à la demande d'examen d'imagerie ;
lorsque la procédure d'acquisition d'images complète précédente a été réalisée dans le délai donné, la détermination permettant de savoir si la raison exige un certain nombre de vues inférieur à un seuil de vues ; et
lorsque le nombre de vues est inférieur au seuil de vues, la génération d'une indication selon laquelle la procédure d'acquisition d'images doit être réalisée en tant que procédure d'acquisition d'images limitée.

2. Procédé selon la revendication 1, dans lequel la procédure d'acquisition d'images correspond à des opérations de capture d'images réalisées à l'aide d'un examen d'échocardiographie, dans lequel l'examen d'échocardiographie est l'un parmi un examen d'échocardiographie complet ou un examen d'échocardiographie limité.

3. Procédé selon la revendication 2, dans lequel l'examen d'échocardiographie complet présente un nombre moyen de vues créées, dans lequel le seuil de vues est un pourcentage du nombre moyen de vues.

4. Procédé selon la revendication 3, dans lequel le seuil de vues est égal à 30 % du nombre moyen de vues.

5. Procédé selon la revendication 1, dans lequel le délai donné est de 1 semaine.

6. Procédé selon la revendication 1, comprenant en outre :
lorsque la procédure d'acquisition d'images doit être réalisée en tant que procédure d'acquisition d'images limitée, la détermination d'au moins une séquence d'un examen d'échocardiographie à utiliser lors de la procédure d'acquisition d'images ; et
la génération d'une indication supplémentaire incluant la au moins une séquence.

7. Procédé selon la revendication 6, dans lequel la au moins une séquence est déterminée en fonction de la raison et d'une correspondance entre une anatomie de la raison et une vue correspondante.

8. Procédé selon la revendication 1, comprenant en outre :
la transmission de l'indication à l'un parmi un dispositif médical, un dispositif d'interprétation d'images, un dispositif de capture d'images ou une combinaison de ceux-ci.

9. Serveur de flux de travail, comprenant :
un émetteur-récepteur communiquant via un réseau de communication, l'émetteur-récepteur étant configuré pour recevoir une demande d'examen d'imagerie pour un patient, la demande d'examen d'imagerie incluant des informations indiquant une raison de réaliser une procédure d'acquisition d'images, dans laquelle la procédure d'acquisition d'images est soit une procédure d'acquisition d'images complète, soit une procédure d'acquisition d'images limitée, dans laquelle la procédure d'acquisition d'images complète permet d'obtenir un plus grand nombre de vues que la procédure d'acquisition limitée ;
une mémoire stockant un programme exécutable ; et
un processeur qui exécute le programme exécutable qui amène le processeur à réaliser des opérations, comprenant :
la détermination permettant de savoir si le patient a déjà subi une procédure d'acquisition d'images complète dans un délai donné par rapport à la demande d'examen d'imagerie ;
lorsque la procédure d'acquisition d'images complète précédente a été réalisée dans le délai donné, la détermination permettant de savoir si la raison exige un certain nombre de vues inférieur à un seuil de vues ; et
lorsque le nombre de vues est inférieur au seuil de vues, la génération d'une indication selon laquelle la procédure d'acquisition d'images doit être réalisée en tant que procédure d'acquisition d'images limitée.

10. Serveur de flux de travail selon la revendication 9, dans lequel la procédure d'acquisition d'images correspond à des opérations de capture d'images réalisées à l'aide d'un examen d'échocardiographie, dans lequel l'examen d'échocardiographie est l'un parmi un examen d'échocardiographie complet ou un examen d'échocardiographie limité.

11. Serveur de flux de travail selon la revendication 10, dans lequel l'examen d'échocardiographie complet présente un nombre moyen de vues créées, dans lequel le seuil de vues est un pourcentage du nombre moyen de vues.

12. Serveur de flux de travail selon la revendication 11, dans lequel le seuil de vues est égal à 30 % du nombre moyen de vues.

13. Serveur de flux de travail selon la revendication 9, dans lequel le délai donné est de 1 semaine.

14. Serveur de flux de travail selon la revendication 9, dans lequel les opérations du processeur comprennent en outre :
lorsque la procédure d'acquisition d'images doit être réalisée en tant que procédure d'acquisition d'images limitée, la détermination d'au moins une séquence d'un examen d'échocardiographie à utiliser lors de la procédure d'acquisition d'images ; et
la génération d'une indication supplémentaire incluant la au moins une séquence.

15. Serveur de flux de travail selon la revendication 14, dans lequel la au moins une séquence est déterminée en fonction de la raison et d'une correspondance entre une anatomie de la raison et une vue correspondante.
